(19) Europäisches Patentamt
European Patent Office
Office européen des brevets

(11) Numéro de publication: **0 458 699 B1**

(12) **FASCICULE DE BREVET EUROPEEN**

(45) Date de publication de fascicule du brevet: **11.01.95**

(51) Int. Cl.[6]: **A61K 31/195**

(21) Numéro de dépôt: **91401322.2**

(22) Date de dépôt: **23.05.91**

(54) **Utilisation du baclofène pour l'obtention de médicaments destinés au traitement de l'angine de poitrine.**

(30) Priorité: **23.05.90 FR 9006437**

(43) Date de publication de la demande:
**27.11.91 Bulletin 91/48**

(45) Mention de la délivrance du brevet:
**11.01.95 Bulletin 95/02**

(84) Etats contractants désignés:
**AT BE CH DE DK ES FR GB GR IT LI LU NL SE**

(56) Documents cités:
**FR-A- 2 393 577**

**BRITISH JOURNAL OF PHARMACOLOGY, vol. 88, no. 3, juillet 1986, pages 659-670; S. GIULIANI et al.: "Differences in cardiovascular responses to peripherally administered GABA as influenced by basal conditions and type of anaesthesia"**

**EUROPEAN JOURNAL OF PHARMACOLOGY, vol. 71, 1981, pages 53-70; N.G. BOWERY et al.: "Bicuculline-insensitive GABA receptors on peripheral autonomic nerve terminals"**

**W. FORTH et al.: "Pharmakologie und Toxikologie", édition 2, 1983, pages 252-253, B.I.-Wissenschaftsverlag, Mannheim, DE**

(73) Titulaire: **ADIR ET COMPAGNIE
1 rue Carle Hébert
F-92415 Courbevoie Cédex (FR)**

(72) Inventeur: **Bousquet, Pascal Pierre
12 rue St Louis
F-67000 Strasbourg (FR)**

EP 0 458 699 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne l'utilisation à une dose comprise entre 0,5 et 7,5 mg du baclofène, de ses isomères optiques et de ses sels physiologiquement acceptables pour l'obtention de médicaments destinés au traitement de l'angine de poitrine.

Le baclofène, composé de formule I :

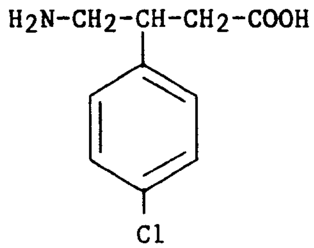

(I)

ou acide 4-amino 3-(4-chlorophényl) butyrique , est un composé décrit dans le brevet Suisse N° 449,046 (1968).

Le baclofène doué d'un pouvoir myorelaxant est utilisé en thérapeutique comme antispastique et notamment dans la contracture spastique de la sclérose en plaques, des affections médullaires ou de la contracture spastique d'origine cérébrale. (Goodman and Gilman's "The pharmacological Basis of therapeutics" $7^{th}$ Ed. Macmillan Publishing Company 1985 p.487).

Il est aussi connu que des compositions pharmaceutiques contenant du baclofène en association avec d'autres médicaments sont utilisables pour le traitement symptomatique de la sclérose multiple (Brevet JP 01319466) ou des affections de la musculature striée (EP 205.492).

Le baclofène, lorsque il est administré en association avec des narcotiques ou des barbituriques, diminue les risques de dépendance ou de l'intoxication qui peuvent survenir de l'utilisation de tels médicaments (Brevet US 4126684).

L'association du baclofène avec des anxiolytiques peut être utile dans le traitement contre l'anxiété (Brevets FR 249 3703 et FR 239 3577).

Il est aussi connu que, lorsque administré conjointement avec un antihypertenseur, le baclofène accroit l'abaissement tensionnel. Le baclofène donc peut être utilisé en association avec différents antihypertenseurs pour le traitement d'hypertension. (Brevet GB 154 7609).

La demanderesse a maintenant trouvé que le baclofène possède d'intéressantes propriétés pharmacologiques applicables à l'obtention des médicaments destinés au traitement de l'angine de poitrine.

En effet, des essais pharmacologiques ont démontré que le baclofène est une substance antiangineuse, parce qu'il prévient l'augmentation de la demande d'oxygène dans les situations déclenchantes de crises d'angine de poitrine, sans déprimer le fonctionnement de base du coeur. Ces situations sont chez l'homme l'effort physique et le stress (Braunwald "Heart Disease, A. textbook of cardiovascular medicine", ED Saunders W.B (1980) pp 1387 - 1389).

Les bases physiopathologiques du traitement de fond de l'angine de poitrine (c'est-à-dire préventive de la crise) consistent en un développement des stratégies thérapeutiques suivantes :

- un accroissement de l'apport d'oxygène au coeur par vasodilatation du lit coronaire, (Parratt, J.R., Pharmacological approaches to the therapy of angina ; Adv. Drug. Res (1974) 8 p 103 - 134)
- une diminution de la consommation d'oxygène par le myocarde en particulier une diminution de l'augmentation de la demande dans les situations déclenchantes de la crise d'angine de poitrine (Sonnenblick Et coll., Am. J. Cardiol. (1968) 22 p 328 - 341).
- une meilleure répartition de l'oxygène vers l'endocarde.

Comme il a été noté, précédemment, le baclofène, diminue la demande d'oxygène.

Au moins deux grandes familles de médicaments déjà existants, s'inscrivent dans ce créneau : les bêta-bloquants et les anticalciques. Cependant, ils affectent tous, le fonctionnement basal du coeur, tant du point de vue de sa force de contraction (inotropisme) que de son rythme de contraction (chronotropisme). De telle sorte qu'ils sont tous potentiellement cardiodépresseurs et/ou arythmogènes. Le baclofène s'inscrit également dans cette stratégie mais selon les résultats des expériences animales, il ne déprime pas le fonctionnement cardiaque de base, ni du point de vue de la force de contraction, ni du point de vue de la fréquence de contraction.

Le composé de formule I est un composé amphotère à caractère plutôt basique, et peut donc être transformé en sel d'addition avec un acide pharmaceutiquement acceptable. Les sels d'additions du composé de formule I font aussi partie de la présente invention. Comme acides, on peut citer l'acide chlorhydrique, sulfurique, bromhydrique, phosphorique etc...

Le composé de formule I ou un de ses sels d'addition peut aussi être associé avec d'autres composés utilisés pour le traitement de l'angine de poitrine, comme par exemple des anticalciques ou des bêta-bloquants.

Les médicaments antiangineux, obtenus en utilisant selon l'invention, le composé de formule I ou ses sels pharmaceutiquement acceptables, sont présentés avantageusement sous des formes diverses telles que par exemple comprimés, dragées, gélules, glossettes ou autres préparations galéniques appropriées pour une administration sublinguale, suppositoires, solutions pour pulvérisations endobuccale, préparations galéniques appropriées pour une administration percutanée, solutions injectables ou buvables.

La posologie peut varier largement en fonction de l'âge du patient, de son poids , la voie et la fréquence d'administration, la gravité et la fréquence des crises de l'angine de poitrine du patient et des traitements associés et s'échelonne de 0,5 à 75 mg par prise ou par application.

Les exemples suivants illustrent l'invention :

**EXAMPLE 1**

**Effet du Baclofène sur les modifications cardiovasculaires dues à une stimulation diencéphalique : Essai N°1**

Dans le cadre d'une étude expérimentale portant sur la production d'un modèle animal des manifestations cardiovasculaires rencontrées lors du stress ou de l'effort physique, il a été possible de reproduire sur l'animal anesthésié, en l'occurence le chat, de telles manifestations par la stimulation électrique d'une zone précise du diencéphale.

Des chats de 1,5 à 3,5 Kg (n = 4) ont été anesthésiés au pentobarbital (30 à 40 mg/kg administrés d'abord par voie i.p., et ensuite 3 à 5 mg/kg par voie i.v.) puis trachéotomisés, curarisés et ventilés artificiellement. Les différents paramètres hémodynamiques de base ont été enregistrés : pressions artérielles systolique et diastolique, fréquence cardiaque, débit cardiaque. Les différents index et paramètres hémodynamiques ont aussi été calculés (pression artérielle moyenne, Dp/dt, double produit fréquence x pression).

La température centrale des animaux a été maintenue entre 37 et 37,5 °C au moyen d'une couverture chauffante. Puis, sur les animaux placés dans un appareil de stéréotaxie, la stimulation électrique de l'aire de défense a été effectuée au moyen d'une électrode placée dans la substance grise, aux coordonnées $A_6$ $L_1$ $H_0$. Les stimulations sont délivrées grâce à un stimulateur fonctionnant en régime monopolaire : fréquence 100 Hz, durée 3 msec, différence de potentiel 3-6 volts.

Le positionnement de l'électrode est considéré comme adéquat lorsque le débit cardiaque et Dp/dt sont majorés de plus de 20 %.

L'administration du baclofène a été effectuée par la veine fémorale. Les animaux ont reçu selon le cas 0,5 mg/kg ou 1 mg/kg. Puis les différents paramètres ont été relevés 15 minutes et 30 minutes après l'injection de baclofène.

Comme les résultats du tableau I le démontrent, la stimulation éléctrique diencéphalique provoque chez l'animal anesthésié, une augmentation de la fréquence cardiaque, de la pression artérielle, du débit cardiaque, du Dp/dt et du travail cardiaque, ce qui concorde avec les résultats de Folkow et Coll (Acta. Physiol. Scand. (1968) 72, p. 220) et Kylstra et Coll (Acta. Physiol. Scand. (1970) 78, p. 386), (J. Pharm. Exp. Ther (1982), 223 p. 654) et Djojosugito et Coll (Acta. Physiol. Scand. (1970) 78, p. 376).

Il est connu que la consommation d'oxygène dépend du travail cardiaque qui lui même dépend de la fréquence cardiaque et de la pression artérielle. La consommation d'oxygène est estimée par le double produit : Fréquence cardiaque X Pression artérielle systolique. La stimulation de l'aire de défense provoque une importante majoration du travail myocardiaque et donc de la consommation d'oxygène.

L'administration de baclofène, bien que ne modifiant en rien les paramètres hémodynamiques de base, inhibe de façon déjà significative certaines réponses hémodynamiques à la stimulation diencéphalique, dès la dose de 0,5 mg/kg et les écrète complétement à la dose de 1 mg/kg ($p < 0.001$).

**T A B L E A U I**

| | SANS BACLOFENE | | | BACOFLENE 0,5 mg/Kg - i.v. | | | BACOFLENE 1 mg/Kg - i.v. | | |
|---|---|---|---|---|---|---|---|---|---|
| | Valeurs de base | Valeurs sous stimulation | Δ | Valeurs de base | Valeurs sous stimulation | Δ | Valeurs de base | Valeurs sous stimulation | Δ |
| Fc | 155 ± 5,5 | 192,5 ± 4 | + 37,5 | 160 ± 3,5 | 172,5 ± 4,2 | + 12,5 | 157,5 ± 4,2 | 160 ± 2,2 | + 2,5 |
| PAS | 158 ± 2 | 197,5 ± 5,5 | + 39,5 | 150 ± 1 | 170 ± 5 | + 20 | 152,5 ± 2,2 | 157,5 ± 2,2 | + 5 |
| Fc x PAS | 24040 ± 996 | 38100 ± 1790 | + 14060 | 23875 ± 446 | 29350 ± 1255 | + 5475 | 24000 ± 530 | 24050 ± 469 | + 50 |
| PAM | 130,5 ± 2,2 | 169 ± 4,9 | + 38,5 | 129 ± 5,6 | 144,5 ± 3,6 | + 15,3 | 132,5 ± 2 | 139 ± 3,8 | + 6,5 |
| Dc | 0,58 ± 0,062 | 0,79 ± 0,06 | + 0,21 | 0,59 ± 0,06 | 0,66 ± 0,062 | + 0,07 | 0,58 ± 0,062 | 0,60 ± 0,060 | + 0,02 |
| Dp/dt | 3465 ± 108 | 4400 ± 244 | + 925 | 3400 ± 122 | 4150 ± 258 | + 750 | 3350 ± 148 | 3425 ± 170 | + 75 |

**Δ** = Différence entre la valeur de base et celle sous stimulation

**Fc** = Fréquence cardiaque (battements par minute)

**Dc** = Débit cardiaque (1/min)

**PAS** = Pression artérielle systolique (mm Hg)

**Fc x PAS** = Double produit fréquence x pression (battements x mm Hg x $min^{-1}$)

**PAM** = Pression artérielle moyenne (mm Hg)

**Dp/Dt** = mm Hg x $s^{-1}$

**EXEMPLE 2**

**Effet du Baclofène sur les modifcations cardiovasculaires dues à une stimulation diencéphalique : Essai N°2**

Le protocole utilisé pour cette étude a été identique à celui utilisé dans l'exemple 1. Le nombre des animaux utilisés (chats) était de 12.

Lors de cette étude, le débit cardiaque n'a pas été mesuré. Les résultats contenus dans le tableau II confirment l'activité cardioprotectrice du baclofène.

T A B L E A U  II

| | SANS BACLOFENE | | | BACOFLENE 0,5 mg/Kg - i.v. | | | BACOFLENE 1 mg/Kg - i.v. | | |
|---|---|---|---|---|---|---|---|---|---|
| | Valeurs de base | Valeurs sous stimulation | Δ | Valeurs de base | Valeurs sous stimulation | Δ | Valeurs de base | Valeurs sous stimulation | Δ |
| **Fc** | 169 ± 3,5 | 200 ± 5 | + 31 | 171 ± 3 | 187 ± 3,5 | + 16 | 168 ± 4 | 173 ± 4 | + 5 |
| **PAS** | 152 ± 7 | 194 ± 8 | + 42 | 152 ± 5 | 176 ± 5 | + 24 | 153 ± 4 | 154 ± 5 | + 1 |
| **Fc x PAS** | 25775 ± 1255 | 38533 ± 2154 | +12758 | 26003 ± 1035 | 32918 ± 1315 | + 6915 | 25841 ± 938 | 26245 ± 1189 | + 404 |
| **PAM** | 123 ± 6,5 | 156 ± 7,5 | + 33 | 119 ± 2,4 | 138 ± 6 | + 19 | 114 ± 4,3 | 122 ± 6 | + 8 |
| **Dp/dt** | 3675 ± 222 | 5033 ± 301 | + 1358 | 3641 ± 211 | 4750 ± 266 | + 1109 | 3566 ± 204 | 3900 ± 213 | + 334 |

Δ = Différence entre la valeur de base et celle sous stimulation
**Fc** = Fréquence cardiaque (battements par minute)
**PAS** = Pression artérielle systolique (mm Hg)
**Fc x PAS** = Double produit fréquence x pression (battements x mm Hg x $min^{-1}$)
**PAM** = Pression artérielle moyenne (mm Hg)
**Dp/Dt** = mm Hg x $s^{-1}$

## Revendications

**Revendications pour les Etats contractants suivants : AT, BE, CH, DE, DK, FR, GB, IT, LI, LU, NL, SE**

**1.** Utilisation à une dose comprise entre 0,5 et 75 mg du baclofène, composé de formule I :

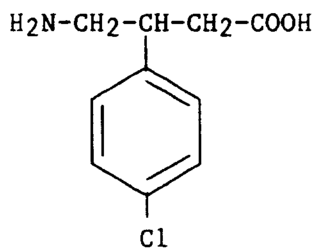

(I)

de ses isomères et de ses sels d'addition avec un acide ou une base pharmaceutiquement acceptable pour l'obtention de médicament déstinés au traitement de l'angine de poitrine.

**2.** Utilisation du baclofène, composé de formule I selon la revendication 1, un de ses isomères ou un de ses sels d'addition avec un acide ou une base minéral ou organique pharmaceutiquement acceptable, en association avec un autre composé utilisable pour le traitement de l'angine de poitrine.

**Revendications pour l'Etat contractant suivant : ES**

**1.** Utilisation à une dose comprise entre 0,5 et 75 mg du baclofène, composé de formule I :

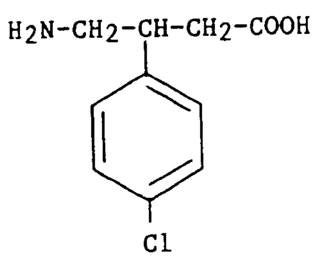

(I)

de ses isomères et de ses sels d'addition avec un acide ou une base pharmaceutiquement acceptable pour l'obtention de médicament déstinés au traitement de l'angine de poitrine.

**2.** Utilisation du baclofène, composé de formule I selon la revendication 1, un de ses isomères ou un de ses sels d'addition avec un acide ou une base minéral ou organique pharmaceutiquement acceptable, en association avec un autre composé utilisable pour le traitement de l'angine de poitrine.

**Revendications pour l'Etat contractant suivant : GR**

**1.** Utilisation à une dose comprise entre 0,5 et 75 mg du baclofène, composé de formule I :

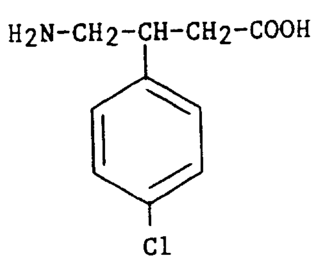

(I)

de ses isomères et de ses sels d'addition avec un acide ou une base pharmaceutiquement acceptable

pour l'obtention de médicament déstinés au traitement de l'angine de poitrine.

**2.** Utilisation du baclofène, composé de formule I selon la revendication 1, un de ses isomères ou un de ses sels d'addition avec un acide ou une base minéral ou organique pharmaceutiquement acceptable, en association avec un autre composé utilisable pour le traitement de l'angine de poitrine.

**Claims**

**Claims for the following Contracting States : AT, BE, CH, DE, DK, FR, GB, IT, LI, LU, NL, SE**

**1.** Use at a dose of from 0.5 to 75 mg of baclofen, the compound of the formula I:

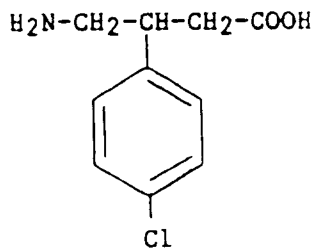

(I)

its isomers and its addition salts with a pharmaceutically acceptable acid or base in the preparation of medicaments for the treatment of angina pectoris.

**2.** Use of baclofen, the compound of the formula I according to claim 1, one of its isomers or one of its addition salts with a pharmaceutically acceptable mineral or organic acid or base, in association with another compound that can be used in the treatment of angina pectoris.

**Claims for the following Contracting State : ES**

**1.** Use at a dose of from 0.5 to 75 mg of baclofen, the compound of the formula I:

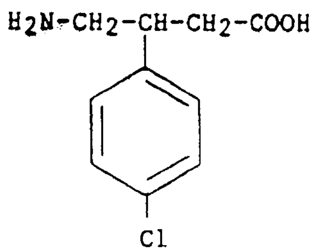

(I)

its isomers and its addition salts with a pharmaceutically acceptable acid or base in the preparation of medicaments for the treatment of angina pectoris.

**2.** Use of baclofen, the compound of the formula I according to claim 1, one of its isomers or one of its addition salts with a pharmaceutically acceptable mineral or organic acid or base, in association with another compound that can be used in the treatment of angina pectoris.

**Claims for the following Contracting State : GR**

**1.** Use at a dose of from 0.5 to 75 mg of baclofen, the compound of the formula I:

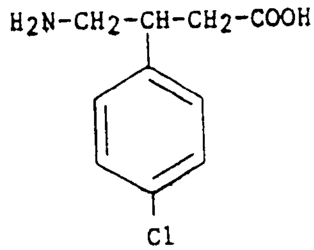

(I)

its isomers and its addition salts with a pharmaceutically acceptable acid or base in the preparation of medicaments for the treatment of angina pectoris.

**2.** Use of baclofen, the compound of the formula I according to claim 1, one of its isomers or one of its addition salts with a pharmaceutically acceptable mineral or organic acid or base, in association with another compound that can be used in the treatment of angina pectoris.

**Patentansprüche**
**Patentansprüche für folgende Vertragsstaaten : AT, BE, CH, DE, DK, FR, GB, IT, LI, LU, NL, SE**

**1.** Verwendung von Baclofen, der Verbindung der Formel I:

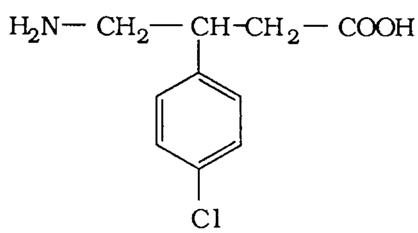

(I)

seiner Isomeren und seiner Additionssalze mit einer pharmazeutisch annehmbaren Säure oder Base in einer Dosis zwischen 0,5 und 75 mg zur Herstellung eines Arzneimittels zur Behandlung von Angina pectoris.

**2.** Verwendung von Baclofen, Verbindung der Formel I nach Anspruch 1, eines seiner Isomeren oder eines seiner Additionssalze mit einer anorganischen oder organischen, pharmazeutisch annehmbaren Säure oder Base in Kombination mit einer weiteren Verbindung, die für die Behandlung der Angina pectoris geeignet ist.

**Patentansprüche für folgenden Vertragsstaat : ES**

**1.** Verwendung von Baclofen, der Verbindung der Formel I:

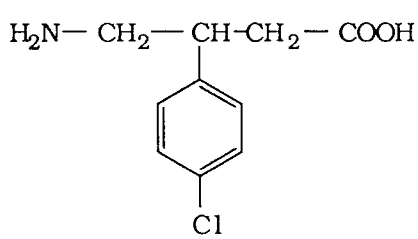

(I)

seiner Isomeren und seiner Additionssalze mit einer pharmazeutisch annehmbaren Säure oder Base in einer Dosis zwischen 0.5 und 75 mg zur Herstellung eines Arzneimittels zur Behandlung von Angina pectoris.

**2.** Verwendung von Baclofen, Verbindung der Formel I nach Anspruch 1, eines seiner Isomeren oder eines seiner Additionssalze mit einer anorganischen oder organischen, pharmazeutisch annehmbaren Säure oder Base in Kombination mit einer weiteren Verbindung, die für die Behandlung der Angina pectoris geeignet ist.

**Patentansprüche für folgenden Vertragsstaat : GR**

**1.** Verwendung von Baclofen, der Verbindung der Formel I:

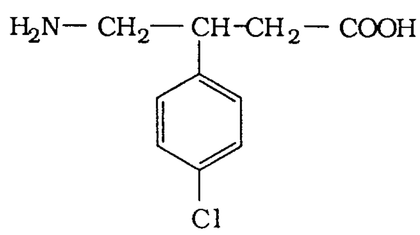

(I)

seiner Isomeren und seiner Additionssalze mit einer pharmazeutisch annehmbaren Säure oder Base in einer Dosis zwischen 0,5 und 75 mg zur Herstellung eines Arzneimittels zur Behandlung von Angina pectoris.

**2.** Verwendung von Baclofen, Verbindung der Formel I nach Anspruch 1, eines seiner Isomeren oder eines seiner Additionssalze mit einer anorganischen oder organischen, pharmazeutisch annehmbaren Säure oder Base in Kombination mit einer weiteren Verbindung, die für die Behandlung der Angina pectoris geeignet ist.